# EUROPÄISCHE PATENTSCHRIFT

(11) **EP 1 334 745 B1**
(45) Veröffentlichungstag und Bekanntmachung des Hinweises auf die Patenterteilung: **17.05.2017**
(21) Anmeldenummer: 03003055.5
(22) Anmeldetag: 12.02.2003
(51) Int. Cl.: A61N 1/05

(54) **Führungsdraht und implantierbare Elektrodenleitung**
Guide wire and implantable lead
Fil de guidage et conducteur implantable

(30) Priorität: 12.02.2002 DE 10205721
(43) Veröffentlichungstag der Anmeldung: 13.08.2003
(73) Patentinhaber: BIOTRONIK SE & Co. KG, 12359 Berlin (DE)
(72) Erfinder: Täubert, Kerstin, 12589 Berlin (DE); Voigt, Siegfried, 12161 Berlin (DE); Flach, Erhard, 12305 Berlin (DE); Kolberg, Gernot, 12043 Berlin (DE); Hahnke, Gerhard, 12623 Berlin (DE); Junge, Agur, 10787 Berlin (DE)

(56) Entgegenhaltungen:
- EP-A- 0 951 920
- US-A- 4 570 642
- US-A- 5 497 782
- US-A- 6 033 414
- US-A1- 2001 037 136

## Beschreibung

Die Erfindung betrifft eine Anordnung aus einer implantierbaren Herzelektrodenleitung zum intrakardialen Abfühlen von Herzaktionspotentialen und/oder zur elektrischen Stimulation oder Defibrillation des Herzens und einem Führungsdraht zur Einführung der Elektrodenleitung in das Herz eines Patienten

Implantierbare Elektrodenleitungen mit den genannten Funktionen sind in großer Vielgestaltigkeit bekannt und seit langem in Verbindung mit implantierten Herzschrittmachern oder Defibrillatoren massenhaft im praktischen Einsatz. Da diese Elektrodenleitungen mit Rücksicht auf den Verlauf des Gefäßsystems, in dem sie sich vom implantierten Gerät in das Herz des Patienten erstrecken, hochgradig flexibel sein müssen, wird für ihre Implantation als Einführwerkzeug ein Führungsdraht benötigt. Um den Führungsdraht aufnehmen zu können, haben die Elektrodenleitungen einen sich in Längsrichtung erstreckenden Hohlraum, das sogenannte Lumen. Mit dem eingesetzten Führungsdraht kann die Elektrodenleitung unter Beobachtung ihres Weges mittels eines büdgebenden Verfahrens zielgenau in das Herz geführt werden, wobei bei Bedarf durch Drehen eines Griffes am proximalen Ende des Führungsdrahtes dessen mit einer Krümmung versehenes distales Ende in die gewünschte Richtung gelenkt werden kann.

Bei den bekannten Implantationsverfahren dieser Art wird der Führungsdraht (Mandrin) bis zum distalen Anschlag in die Elektrodenleitung eingeschoben. Dadurch wird die Elektrode gegebenenfalls gestreckt und erhält einen Verlauf und eine Steifigkeit, die das Einführen in das Herz durch das (venöse) Gefäßsystem erst ermöglichen - was jedoch den zuverlässigen Verbleib des Führungsdrahtes im wesentlichen in der Gesamterstreckung des Lumens der Elektrodenleitung voraussetzt. Infolge der vielgestaltigen Manipulationen des Implanteurs kommt es jedoch bei den herkömmlichen Anordnungen relativ häufig zu unbeabsichtigten Verschiebungen und unter Umständen zu einem Herausrutschen des Führungsdrahtes aus dem Lumen der Elektrodenleitung.

Aus der US 5 497 782 ist ein verriegelbarer Führungsdraht zum Einsetzen und Austauschen eines Dilatationskatheters bekannt. Hier dient ein spiralig aufweitbarer Abschnitt des Führungsdrahtes zur dessen zuverlässiger Positionierung bezüglich einer Läsion, wenn der Dilatationskatheter herausgezogen und durch einen neuen ersetzt wird.

Aus der US 6 027 461 ist ein Infusions-Führungsdraht mit festgelegtem Kerndraht bekannt. Das proximale Ende dieses Kerndrahtes ist an einem proximalen Verbindergehäuse zum Anschluss eines Infusionslumens fixiert. Dieses Dokument ist als nächstliegender Stand der Technik anzusehen Die US-A-4570642 offenbart eine Anordnung für die Einführung und den Austausch eines Ballon-Katheters, welche aus einem Führungskatheter und einem verriegelbaren Führungsdraht besteht.

Die Aufgabe der Erfindung besteht darin, eine verbesserte Anordnung der genannten Art anzugeben, die eine sichere und schnelle Implantation auch unter widrigen Umständen, beispielsweise bei sehr ungünstigem geometrischem Verlauf des Gefäßsystems und der entsprechenden Notwendigkeit komplizierter Handhabungen seitens des Implanteurs, gewährleisten.

Diese Aufgabe wird gemäß einem ersten Aspekt der Erfindung durch eine Anordnung mit den Merkmalen des Anspruchs 1 und gemäß einem zweiten Aspekt durch eine Anordnung mit den Merkmalen des Anspruchs 3 gelöst.

Die Erfindung schließt den grundlegenden Gedanken des Vorsehens einer gegenseitigen lösbaren Verriegelung zwischen Führungsdraht und Elektrodenleitung zur Festlegung des Führungsdrahtes im Lumen der Elektrodenleitung während des Implantationsvorganges ein. Sie schließt weiter den Gedanken ein, eine derartige Festlegung bzw. Verriegelung am proximalen Ende der Anordnung vorzusehen, um sie nach Beendigung der Implantation möglichst leicht wieder lösen zu können. Anzumerken ist dabei, dass die vorgeschlagene Fixierung nicht notwendigerweise voraussetzt, dass der Führungsdraht vollständig bis ins distale Ende des Elektrodenleitungs-Lumens eingeschoben ist.

In einer Variante der Erfindung ist als Verriegelungsmittel eine den effektiven Kontaktdurchmesser des Führungsdrahtes auf einen im wesentlichen dem Innendurchmesser eines Lumens der Elektrodenleitung entsprechenden oder diesem gegenüber größeren Wert vergrößernde, insbesondere bogen-, wellen-, V-, zickzack- oder trapezförmige, Verformung vor. Die hochgradige Elastizität des Materials des Führungsdrahtes in Verbindung mit dieser eingeprägten Verformung führt dazu, dass der Führungsdraht punktuell (an einer Stelle oder mehreren Stellen) elastisch gegen die Wandung des Lumens angepresst wird, wodurch ein Reibschluss mit der Elektrodenleitung und damit die gewünschte Arretierung bewirkt wird. Die genannte Verformung stellt ein besonders einfaches Fixierungsmittel dar.

Gemäß dem zweiten Aspekte der Erfindung hat die mit einem Lumen zur Aufnahme des Führungsdrahtes versehene Elektrodenleitung an ihrem proximalen Ende einen Steckerstift, und der Führungsdraht weist eine Klemmhülse zur gegenseitigen lösbaren Verriegelung des Führungsdrahtes mit der Elektrodenleitung auf, wobei die Klemmhülse zwei in Längsrichtung aneinander anschließende, zueinander exzentrisch angeordnete Längsbohrungen aufweist, durch welche der Führungsdraht läuft, wobei der Durchmesser der distalen Längsbohrung auf den Außendurchmesser des Steckerstiftes der Elektrodenleitung derart abgestimmt ist, dass der Steckerstift in der distalen Längsbohrung der Klemmhülse durch Reibschluss fixiert ist und damit ein kraftschlüssiger Eingriff zwischen Elektrodenleitung und Klemmhülse entsteht, wobei die proximale und distale Längsbohrung der Klemmhülse zueinander derart axial versetzt sind, dass beim Aufschieben der Klemmhülse mit ihrer distalen Längsbohrung auf den Steckerstift eine Auslenkung des Führungsdrahtes entlang seiner Längserstreckung erfolgt, die ein Anpressen des Führungsdrahtes gegen die Wand der proximalen Längsbohrung verursacht und somit einen kraftschlüssigen Eingriff zwischen Führungsdraht und Klemmhülse erzeugt.

Vorteile und Zweckmäßigkeit der Erfindung ergeben sich im übrigen aus den abhängigen Ansprüchen sowie der nachfolgenden, skizzenartigen Beschreibung beispielhafter Anordnungen anhand der Figuren. Von diesen zeigen:
- Fig. 1A und 1B: skizzenartige perspektivische Darstellungen einer erfindungsgemäßen Anordnung,
- Fig. 2A und 2B: skizzenartige perspektivische Darstellungen einer nicht erfindungsgemäßen Anordnung,
- Fig. 3A und 3 B: skizzenartige perspektivische Darstellungen einer weiteren nicht erfindungsgemäßen Anordnung,
- Fig. 4A bis 4C: schematische Darstellungen (Längsschnittdarstellungen bzw. Vorderansicht der Klemmhülse) einer Ausführungsform der erfindungsgemäßen Anordnung.

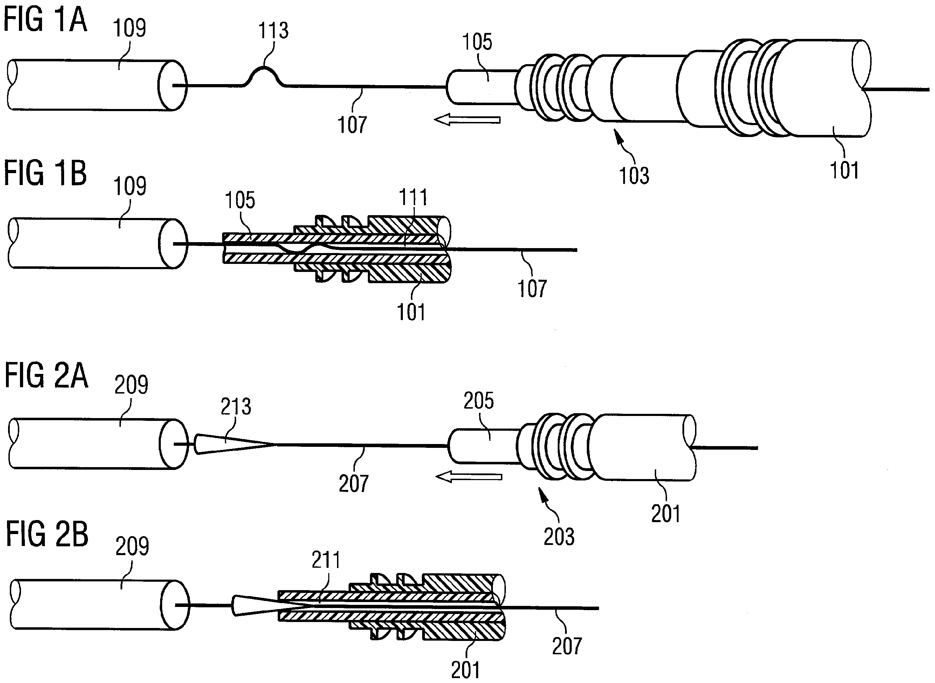

Fig. 1A und 1B zeigen in (bei Fig. 1B teilweise geschnittenem) skizzenartigen perspektivischen Darstellungen das proximale Ende einer implantierbaren Elektrodenleitung 101 mit einem mehrpoligen Stecker 103, der einen Steckerstift 105 umfasst, mit einem eingeführten Führungsdraht 107 mit Griffstück 109. In Fig. 1B ist zu erkennen, dass die Elektrodenleitung 101 - einschließlich des Steckers 103 - ein zentrales Lumen 111 hat, durch das der Führungsdraht 107 hindurchgeht.

Nahe dem proximalen Ende des Führungsdrahtes 107, kurz vor dessen Einmündung in das Griffstück 109, hat der Führungsdraht eine bogenförmige Verformung 113, die seinen Wirkdurchmesser auf einen Wert oberhalb des Innendurchmessers des Lumen 111 vergrößert. Wie Fig. 1B zeigt, findet bei Einschieben des Führungsdrahtes in das Lumen 111 bis über die Position der Verformung 113 hinaus eine wellenförmige elastische Deformation des entsprechenden Abschnittes des Führungsdrahtes statt. Dies führt zu einer elastischen Anpressung des Führungsdrahtes an mehreren Punkten der Innenwandung des Lumens 111, wodurch ein Reibschluss zwischen dem Führungsdraht 107 und der Elektrodenleitung 101 erzeugt wird.

Bei geeigneter, auf die Elastizitätseigenschaften des Führungsdrahtes und den Reibbeiwert zwischen Führungsdraht und Lumenwandung abgestimmten Gestaltung der Verformung 113 reicht die Reibungskraft aus, um den Führungsdraht bei allen Handhabungen im Zusammenhang mit der Einführung der Elektrodenleitung in das Herz eines Patienten auftretenden Manipulationen sicher in der Elektrodenleitung zu halten. Nach Beendigung der Implantation kann der Führungsdraht 107 am Griffstück 109 bei festgehaltener Elektrodenleitung 101 im Bereich des Steckers 103 unter Überwindung dieser Reibungskraft wieder aus der Elektrodenleitung herausgezogen werden.

In Fig. 2A und 2B ist eine weitere Anordnung aus einer Elektrodenleitung 201 mit einem Stecker 203 sowie einem zugehörigen Steckerstift 205 und einem Führungsdraht 207 mit Griffstück 209 dargestellt. Hier ist zur Fixierung des Führungsdrahtes in einem Lumen 211 der Elektrodenleitung 201 ein kegelförmiger Aufsatz 213 auf dem Führungsdraht 207 in unmittelbarer Nachbarschaft zur distalen Stirnfläche des Griffstücks 209 vorgesehen. Wie in Fig. 2 zu erkennen ist, wird beim Einführen des Führungsdrahtes in das Lumen der Elektrodenleitung der Kegel 213 - der bevorzugt aus einem Material mit einem hohen Reibungskoeffizienten gegenüber dem Material der Wandung des Lumens 211 besteht - teilweise in das Lumen hineingeschoben. Er wird dabei gegen den äußersten proximalen Abschnitt der Lumenwandung angepresst, und es entsteht auch hier ein Reibschluss, der (geeignete Materialwahl und Dimensionierung vorausgesetzt) den Führungsdraht 207 bei allen Manipulationen sicher in der Elektrodenleitung 201 hält.

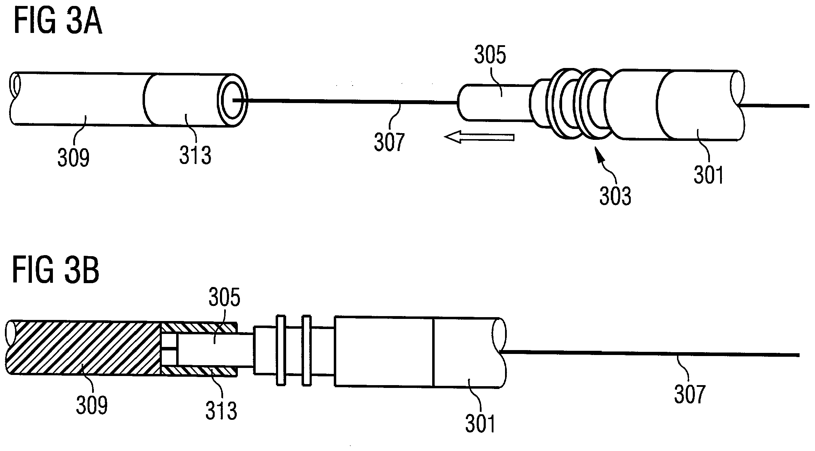

Als weitere Variante ist in Fig. 3A und 3B - wiederum skizzenartig - eine weitere Anordnung aus einer Elektrodenleitung 301 mit Steckerbereich 303 und Steckerstift 305 und einem Führungsdraht 207 mit Griffstück 309 dargestellt. Hier ist ein ringförmiger Elastomer-Fortsatz 313 am distalen Ende des Griffstückes 309 als ein über Kraftschluss wirkendes Verriegelungselement zwischen Elektrodenleitung und Führungsdraht vorgesehen.

Wie in Fig. 3B verdeutlicht wird, umschließt der Elastomer-Fortsatz (z. B. Gummiring) 313 den Umfang des Steckerstiftes 305 und übt auf diesen eine elastische Anpresskraft aus. Diese ist durch geeignete Materialwahl und Dimensionierung beider Teile wieder derart einstellbar, dass der Führungsdraht für die Dauer des Implantationsvorganges sicher in der Elektrodenleitung gehalten wird und gleichwohl nach Beendigung des Einführens relativ leicht wieder von dieser zu lösen ist. Anstelle eines Elastomeren kann hier auch ein relativ hartes Thermoplastteil eingesetzt werden.

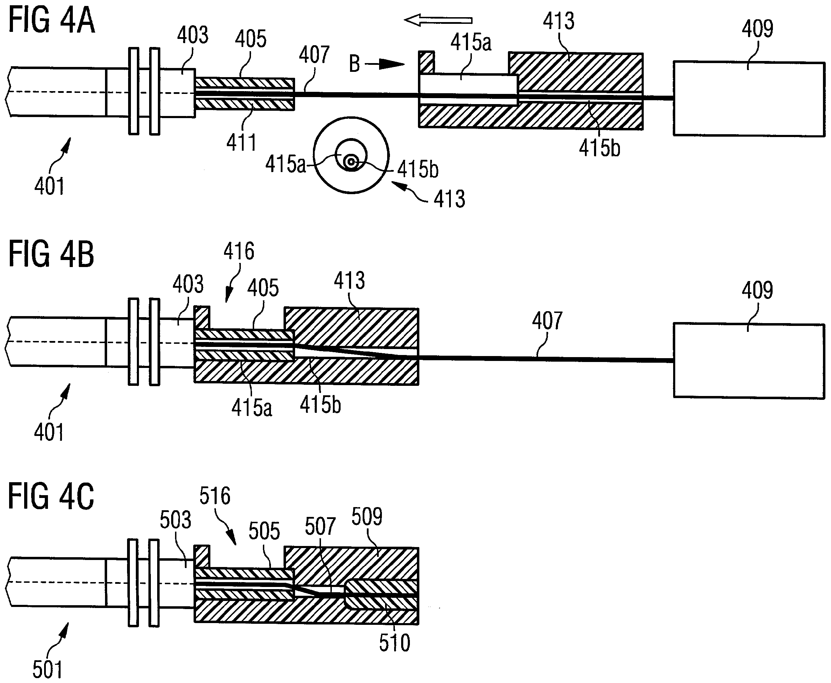

In Fig. 4A und 4B ist eine Ausführung der Erfindung schematisch dargestellt, bei der eine Elektrodenleitung 401 mit Steckerbereich 403 und Steckerstift 405 auf einen Führungsdraht 407 aufgesteckt ist, der ein Griffstück 409 hat. Hier ist als Arretierungselement zwischen Elektrodenleitung 401 und Führungsdraht 407 eine separate, auf dem Führungsdraht verschiebliche Klemmhülse 413 vorgesehen, die zwei in Längsrichtung aneinander anschließende Längsbohrungen 415a, 415b mit unterschiedlichem Durchmesser und zueinander exzentrischer Anordnung hat.

Wie Fig. 4B schematisch zeigt, wird um in ein Lumen 411 der Elektrodenleitung 401 eingeführten Zustand des Führungsdrahtes 407 beim Aufschieben der Klemmhülse 413 mit ihrer distalen Längsbohrung 415a auf den Steckerstift 405 eine Auslenkung des Führungsdrahtes in dessen Längserstreckung bewirkt. Diese führt zu einem Anpressen desselben gegen die Wand der Bohrung 415b nahe deren distalen und proximalen Ende. Da die Klemmhülse 413 zugleich hinsichtlich Ihrer Abmessungen und Materialwahl derart ausgebildet ist, dass auch der Steckerstift 405 in der Bohrung 415a durch Reibschluss fixiert ist, ergibt sich insgesamt eine Fixierung zwischen Führungsdraht und Elektrodenleitung. Diese verhindert ein Herausrutschen des ersteren aus der letzteren infolge von Manipulationen beim Implantationsvorgang. Im Bereich der distalen Bohrung 415a hat die Klemmhülse 413 ein Anschlussfenster 416, über das erforderlichenfalls der Steckstift 405 von außen elektrisch kontaktiert werden kann.

Ist während der Implantation der Elektrodenleitung 401 die richtige Position für den Führungsdraht gefunden, wird die Klemmhülse 413 auf den Stecker 403 geschoben, wobei der Steckerstift 405 in die distale Bohrung 415a der Klemmhülse eindringt. Aufgrund des axialen Versatzes der Bohrungen 415a, 415b in der Klemmhülse wird dabei die erwähnte Auslenkung bzw. Schrägstellung des Führungsdrahtes 407 erzeugt, die zur Arretierung zwischen Führungsdraht und Elektrodenleitung führt. Die Elektrodenleitung kann nun weiter vorgeschoben werden, ohne dass der Führungsdraht seine Position relativ zu ihr ändern kann. Nach erfolgter Implantation wird wieder die Klemmhülse 413 vom Elektrodenstecker 403 abgezogen, und dann lässt sich ohne weiteres auch der Führungsdraht 407 am Griffstück 409 wieder aus der Elektrodenleitung herausziehen.

In Fig. 4C ist eine modifizierte Realisierung des letztgenannten Funktionsprinzips mit einer Elektrodenleitung 501 (mit Stecker 503 und Steckerstift 505 /übereinstimmend mit der Ausführung nach Fig. 4A und 4B) und einem Führungsdraht 507 mit einem zugleich als Klemmhülse wirkenden Griffstück 509 dargestellt.

Das Griffstück 509 hat einen weitgehend mit dem Aufbau der Klemmhülse 413 nach Fig. 4A bis 4C übereinstimmenden Aufbau, wobei der Führungsdraht in einem Fixierungsstopfen 510 gehalten ist, der in das proximale Ende des Griffstückes integriert ist. Auch hier ist ein (mit 516 bezeichnetes Anschlussfenster 516 vorgesehen).

### Bezugszeichenliste

- 101; 201; 301; 401; 501: Elektrodenleitung
- 103; 203; 303; 403; 503: Stecker
- 105; 205; 305; 405; 505: Steckerstift
- 107; 207; 305; 407; 507: Führungsdraht
- 109; 209; 309; 409; 509: Griffstück
- 111; 211; 311; 411: Lumen
- 113: Verformung
- 213: kegelförmiger Aufsatz (Kegel)
- 313: Elastomer-Fortsatz
- 413: Klemmhülse
- 415a; 415b: Längsbohrung
- 416; 516: Anschlussfenster

## Patentansprüche

1. Anordnung aus einer implantierbaren Herzelektrodenleitung (101) zum intrakardialen Abfühlen von Herzaktionspotentialen und/oder zur elektrischen Stimulation oder Defibrillation des Herzens und einem Führungsdraht (107) zur Einführung der Elektrodenleitung in das Herz eines Patienten, wobei der Führungsdraht Mittel zur gegenseitigen lösbaren Verriegelung seines proximalen Endes mit der Elektrodenleitung an dem proximalen Ende der Anordnung aufweist, wobei die Verriegelungsmittel eine Verformung (113) aufweisen, die durch punktuelles elastisches Anpressen gegen die Wandung eines Lumens (111) der Elektrodenleitung einen Reibschluss mit einer Reibungskraft erzeugt, um den Führungsdraht bei allen im Zusammenhang mit der Einführung der Elektrodenleitung auftretenden Manipulationen sicher in der Elektrodenleitung zu halten, jedoch das Lösen der Verriegelung nach Beendigung der Implantation zu ermöglichen.

2. Anordnung nach Anspruch 1, wobei die Verformung des Führungsdrahtes bogen-, wellen-, V-, zickzack- oder trapezförmig ist.

3. Anordnung aus einer implantierbaren Herzelektrodenleitung (401; 501)zum intrakardialen Abfühlen von Herzaktionspotentialen und/oder zur elektrischen Stimulation oder Defibrillation des Herzens und einem Führungsdraht (407; 507) zur Einführung der Elektrodenleitung in das Herz eines Patienten,
wobei die mit einem Lumen (411) zur Aufnahme des Führungsdrahtes versehene Elektrodenleitung (401; 501) an ihrem proximalen Ende einen Steckerstift (405; 505) aufweist und
der Führungsdraht eine Klemmhülse (413; 509) zur gegenseitigen lösbaren Verriegelung des Führungsdrahtes mit der Elektrodenleitung aufweist,
wobei die Klemmhülse zwei in Längsrichtung aneinander anschließende, zueinander exzentrisch angeordnete Längsbohrungen (415a, 415b) aufweist, durch welche der Führungsdraht läuft,
wobei der Durchmesser der distalen Längsbohrung (415a) auf den Außendurchmesser des Steckerstiftes der Elektrodenleitung derart abgestimmt ist, dass der Steckerstift (405; 505) in der distalen Längsbohrung der Klemmhülse durch Reibschluss fixiert ist und damit ein kraftschlüssiger Eingriff zwischen Elektrodenleitung und Klemmhülse entsteht,
wobei die proximale und distale Längsbohrung der Klemmhülse zueinander derart axial versetzt sind, dass beim Aufschieben der Klemmhülse mit ihrer distalen Längsbohrung auf den Steckerstift eine Auslenkung des Führungsdrahtes (407) entlang seiner Längserstreckung erfolgt, die ein Anpressen des Führungsdrahtes gegen die Wand der proximalen Längsbohrung (415b) verursacht und somit einen kraftschlüssigen Eingriff zwischen Führungsdraht und Klemmhülse erzeugt.

4. Anordnung nach Anspruch 3,
**dadurch gekennzeichnet, dass**
die Klemmhülse (413) verschiebbar aufgesetzt ist.

5. Anordnung nach Anspruch 3,
**dadurch gekennzeichnet, dass**
die Klemmhülse (509) an einer am proximalen Ende des Führungsdrahtes (507) vorgesehenen Handhabe angeformt oder mit dieser fest verbunden ist.

## Claims

1. An arrangement made of an implantable cardiac electrode lead (101) for intracardiac sensing of cardiac action potentials and/or for electrical stimulation or defibrillation of the heart, and a guide wire (107) for insertion of the electrode lead into the heart of a patient, wherein
the guide wire has, on the proximal end of the arrangement, means to lock the proximal end of the guide wire and the electrode lead with one another in a detachable manner, the means of locking having a deformation (113) that presses in an elastic manner against certain points on the wall of a lumen (111) of the electrode lead to produce a frictional engagement with a frictional force to hold the guide wire securely in the electrode lead during all manipulations that occur in connection with the insertion of the electrode lead, however to allow the locking to be undone after the end of the implantation.

2. The arrangement according to claim 1, wherein the deformation of the guide wire is arc-shaped, wave-shaped, V-shaped, zigzag-shaped, or trapezoidal.

3. An arrangement made of an implantable cardiac electrode lead (401; 501) for intracardiac sensing of cardiac action potentials and/or for electrical stimulation or defibrillation of the heart, and a guide wire (407; 507) for insertion of the electrode lead into the heart of a patient,
wherein the electrode lead (401; 501) with a lumen (411) to hold the guide wire has a plug pin (405; 505) on its proximal end; and
the guide wire has a clamping sleeve (413; 509) to lock the guide wire and the electrode lead with one another in a detachable manner, the clamping sleeve having two longitudinal holes (415a, 415b) that follow one another in the longitudinal direction and that are arranged eccentrically to one another, through which the guide wire runs,
the diameter of the distal longitudinal hole (415a) being matched with the outside diameter of the plug pin of the electrode lead in such a way that the plug pin (405; 505) is fixed in the distal longitudinal hole of the clamping sleeve by frictional engagement, thus producing a frictional engagement between the electrode lead and the clamping sleeve,
the proximal and distal longitudinal holes of the clamping sleeve being axially offset to one another in such a way that when the clamping sleeve with its distal longitudinal hole is pushed onto the plug pin, the guide wire (407) is deflected along its longitudinal extension, which causes the guide wire to press against the wall of the proximal longitudinal hole (415b), and thus produces a frictional engagement between the guide wire and the clamping sleeve.

4. The arrangement according to claim 3, **characterized in that** the clamping sleeve (413) is put on so that it is movable.

5. The arrangement according to claim 3, **characterized in that** the clamping sleeve (509) is formed on a handle provided on the proximal end of the guide wire (507) or solidly connected with the handle.

## Revendications

1. Agencement constitué d'une ligne d'électrode cardiaque (101) implantable pour la détection intracardiaque de potentiels d'action cardiaques et/ou pour la stimulation ou la défibrillation du coeur, et d'un fil de guidage (107) pour l'introduction de la ligne d'électrode dans le coeur d'un patient, où le fil de guidage présente des moyens pour le verrouillage mutuel amovible de son extrémité proximale avec la ligne d'électrode à l'extrémité proximale de l'agencement, où les moyens de verrouillage présentent une déformation (113) qui génère, par une pression élastique ponctuelle contre la paroi d'une lumière (111) de la ligne d'électrode, une liaison par friction avec une force de frottement, afin de maintenir de manière sécurisée le fil de guidage pour toutes les manipulations se produisant en liaison avec l'insertion de la ligne d'électrode dans la ligne d'électrode, en permettant toutefois la libération du verrouillage après la fin de l'implantation.

2. Agencement selon la revendication 1, dans lequel la transformation du fil de guidage est en forme de courbes, d'ondulations, de V, de zigzags ou de trapèzes.

3. Agencement constitué d'une ligne d'électrode cardiaque (401; 501), implantable pour la détection intracardiaque de potentiels d'action cardiaques et/ou pour la stimulation électrique ou la défibrillation du coeur, et d'un fil de guidage (407 ; 507) pour l'insertion de la ligne d'électrode dans le coeur d'un patient, où la ligne d'électrode (401 ; 501) munie d'un lumen (411) pour l'admission d'un fil de guidage présente à son extrémité proximale un connecteur (405 ; 505) et
le fil de guidage présente une douille de serrage (413 ; 509) pour le verrouillage mutuel amovible du fil de guidage avec la ligne d'électrode,
où la douille de serrage présente deux alésages longitudinaux (415a, 415b) se faisant suite l'un à l'autre dans la direction longitudinale, disposés de manière excentrique l'un par rapport à l'autre, lesquels sont traversés par le fil de guidage,
où le diamètre de l'alésage longitudinal distal (415a) est ajusté au diamètre extérieur du connecteur de la ligne d'électrode de telle manière que le connecteur (405 ; 505) est fixé dans l'alésage longitudinal distal de la douille de serrage par une liaison par friction et qu'ainsi une prise par complémentarité des matières est réalisée entre la ligne d'électrode et la douille de serrage,
où les alésages longitudinaux proximal et distal de la douille de serrage sont décalés axialement l'un par rapport à l'autre de telle manière que, lors de l'ouverture par coulissement de la douille de serrage avec son alésage longitudinal distal sur le connecteur, une déviation du fil de guidage (407) se produit le long de son extension longitudinale qui provoque une compression du fil de guidage contre la paroi de l'alésage longitudinal proximal (415b) et génère ainsi une prise par complémentarité des matières entre le fil de guidage et la douille de serrage.

4. Agencement selon la revendication 3, **caractérisé en ce que** la douille de serrage (413) est mise en place en pouvant coulisser.

5. Agencement selon la revendication 3, **caractérisé en ce que** la douille de serrage (509) est moulée sur une poignée prévue à l'extrémité proximale du fil de guidage (507) ou est solidement reliée avec celui-ci.
